(19) Europäisches Patentamt
European Patent Office
Office européen des brevets

(11) EP 0 761 688 A2

(12) EUROPEAN PATENT APPLICATION

(43) Date of publication:
12.03.1997 Bulletin 1997/11

(51) Int Cl.6: **C07K 16/24**, A61K 39/00, A61K 39/395

(21) Application number: **96306164.3**

(22) Date of filing: **23.08.1996**

(84) Designated Contracting States:
**AT BE CH DE DK ES FI FR GB GR IE IT LI LU MC NL PT SE**

(30) Priority: **26.08.1995 GB 9517538**

(71) Applicant: **THERAPEUTIC ANTIBODIES INC.**
**Nashville, Tennessee 37212 (US)**

(72) Inventors:
- **Landon, John, Therapeutic Antibodies, Inc.**
**West Smithfield, London EC1A 7BE (GB)**
- **Smith, Damon Charles,**
**Therapeutic Antibodies, Inc.**
**West Smithfield, London EC1A 7BE (GB)**

(74) Representative: **Bassett, Richard Simon**
**ERIC POTTER & CLARKSON**
**St. Mary's Court**
**St. Mary's Gate**
**Nottingham NG1 1LE (GB)**

# (54) Production, and therapeutic combinations, of antibodies

(57) A method of producing a mixture of polyclonal antibodies, the mixture including polyclonal antibodies which bind to at least two cytokines comprising the steps of (1) administering to an animal a sufficient amount of each a cytokine immunogen to provoke an immune response to each of the cytokine immunogens; (2) allowing an immune response to develop to each of the cytokine immunogens; and (3) removing blood from the animal.

The mixtures of polyclonal antibodies produced are useful in treating shock and shock-like conditions.

A method of preventing or ameliorating septic shock or a shock-like condition in a patient comprising administering to the patient an antibody reactive towards TNFα and an antibody reactive towards IL-1β in a therapeutically effective combination.

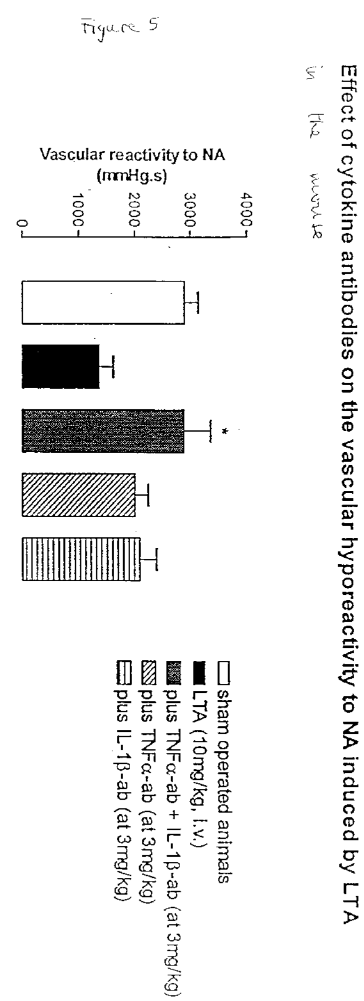


EP 0 761 688 A2

## Description

The present invention relates to the production of antibodies, in particular to the production of polyclonal antibodies directed at two or more cytokines; it further relates to the use of combinations of anti-TNFα and anti-IL-1β antibodies to treat shock and shock-related conditions.

Anti-cytokine polyclonal antibodies are currently produced by immunizing an animal with a single immunogen (such as a purified protein) and, if necessary, boosting the immune response by further administration of the immunogen.

Anti-cytokine polyclonal antibodies (or fragments thereof) are useful in immunotherapy (for example see WO 94/29347) where they neutralize the effect of a particular molecule to which they bind. For example, systemic inflammatory response syndrome (SIRS) which includes septic shock caused by bacteraemia is associated with a rise in the serum concentration of tumour necrosis factor alpha (TNFα).

Polyclonal anti-TNFα Fab fragments have been used to counter the effect of serum TNFα in humans with shock-like conditions (as described in WO 94/29347).

Septic shock, regardless of its aetiology, can be defined as a systemic response to infection resulting in hypotension, which despite adequate fluid resuscitation, results in perfusion abnormalities (American College of Chest Physicians/Society of Critical Care Medicine Consensus Conference (1992) *Crit. Care Med.* **20**, 864). Septic shock, along with sepsis, adult respiratory distress syndrome (ARDS), multi-organ failure and disseminated intravascular coagulopathy may also be defined within the context of a broader definition of acute inflammatory conditions, SIRS (Bone *et al* (1992) *Chest* **101**, 1644). SIRS, therefore, may be caused by a variety of insults and does not require that a site of infection is detected.

The incidence of SIRS in the United States increased by 139% in the period 1979-1987 (74/100,000 to 176/100,000 discharged hospital patients; Bannerjee *et al* (1991) *Am. J. Med.* **91**, 72S) and it has been postulated that this marked increase has been caused by: (i) improved life support technology which keeps intensive care patients with high risk of infection alive for more prolonged periods; (ii) the prevalence of immuno-compromised patients due to an increased incidence of AIDS or chemotherapy and immunotherapy; and (iii) the increased use of invasive medical procedures. Thus, despite the considerable progress made in the management of critically ill patients the reported mortality rates for septic shock (of Gram-positive and Gram-negative origin) remain very high, ranging from 25-75% (Packer & Parillo (1983) *JAMA* **25**, 3324; Snell & Parillo (1991) *Chest* **99**, 1000).

SIRS, whether or not the result of bacteraemia, is associated with a rise in the serum concentration of immunomodulatory cytokines (Friedland *et al* (1992) *Infect. Immun.* **60**, 2402).

However, certain SIRS conditions are associated with a rise in the serum concentration of more than one component. Thus, in recent years a number of additional cytokines (other than TNFα) have been implicated in the aetiology of sepsis and elevated serum levels of IL-1β and IL-6 have been demonstrated in some patients. Some patients have been found to have increased levels of IL-8 (Halstensen *et al* (1993) *J. Infect. Dis.* **167**, 471; Solomkin *et al* (1994) *Infect. Immun.* **62**, 943; Waage *et al* (1994) *Br. J. Haematol.* **86**, 36).

The concentration of these molecules, however, may or may not be related to the severity of the condition.

Cross *et al* (1993) *J. Infect. Dis.* **167**, 112-118 have proposed single, double and triple combination immunotherapy using one or more of an anti-TNFα monoclonal antibody, an *anti-Pseudomonas* O serotype monoclonal antibody and an *anti-Escherichia coli* J5 polyclonal antibody in experimental *Pseudomonas* sepsis. Redmond *et al* (1991) *Ann. Surg.* **214**, 502-509 have used a mixture of anti-IFNγ and anti-IL-4 monoclonal antibodies; and in WO 93/11793 it is disclosed that a combination of anti-IL-6 and anti-TNF antibodies leads to an *increase* in mortality compared to the use of anti-TNF antibody alone, and that a combination of anti-TNF antibody and IL-6 *per se* is preferred.

Russell & Thompson (1993) *Curr. Op. Biotech.* **4**, 714-721 note that IL-1β is not routinely detected in the circulation in clinical sepsis and that high levels of IL-1β may be correlated with survival rather than mortality.

Cerami *et al* (1992) *Clin. Immunol. Immunopath* **62**, S3-S10 notes that in experimental bacteraemia, a large number of cytokines, as well as eicosanoids, platelet activation factor, and a number of reactive oxygen intermediates are induced.

Dinarello *et al* (1993) *JAMA* **269**, 1829-1835 discusses anti-cytokine strategies in the treatment of SIRS but does not disclose the use of combinations of anti-TNFα and anti-IL-1β antibodies.

St John & Dorinsky (1993) *Chest* **103**, 932-943 discuss immunologic therapy for septic shock but does not disclose the use of combinations of anti-TNFα and anti-IL-1β antibodies.

WO 94/10980 discusses the use of encapsulated anti-TNFα and anti-IL-1β monoclonal antibodies in rats challenged with bacteria. No significant difference was observed in survival between animals treated with unencapsulated, neutralizing monoclonal antibodies, antibiotics alone, or unencapsulated neutralizing monoclonal antibodies and antibiotics, compared to no treatment.

Monoclonal antibodies have been shown to lack efficacy in human models of SIRS, for example, the difference in mortality among shock patients treated with placebo or TNF-α mAb was not significant according to Abraham *et al* (1995) *JAMA* **273**, 934-940.

There is, therefore, a need for improved medicines to treat SIRS and similar conditions, and improved processes for making such medicines.

It is an object of the present invention to provide a method which produces mixtures of polyclonal anti-cytokine antibodies.

It is a further object of the present invention to provide combinations of anti-TNFα and anti-IL-1β antibodies and uses thereof.

In this specification "TNF" means tumour necrosis factor, "IL" means interleukin, and "IFN" means interferon.

**Summary of invention**

A first aspect of the invention provides a method of producing a mixture of polyclonal antibodies, the mixture including polyclonal antibodies which bind to at least two cytokines, comprising the steps of:

(1) administering to an animal a sufficient amount of each of a cytokine immunogen to provoke an immune response to each of the cytokine immunogens;
(2) allowing an immune response to develop to each of the cytokine immunogens; and
(3) removing blood from the animal.

By "cytokine immunogen" we mean a molecule which, when administered to an animal, gives rise to the production of antibodies which bind to a cytokine. Preferably, the antibodies bind to the cytokine in the form that the cytokine is found in nature. Thus, the cytokine immunogen is preferably intact, native cytokine. However, the term "cytokine immunogen" also includes variants, fragments and fusions of a cytokine or cytokine mimics which are immunogenic.

The skilled person can readily determine whether an amount of each cytokine immunogen is sufficient to provoke an immune response by determining whether anti-cytokine antibodies appear in the blood of the animal after a suitable period of time, such as several weeks, following administration. Preferably between 40 μg and 200 μg of each cytokine immunogen per animal (such as a sheep) is used; more preferably about 50μg of each cytokine immunogen is used. An immune response to the cytokine immunogen can usually be seen two weeks after the initial immunisation; six to twenty weeks from the initial immunisation is usually required for the maximum response.

The cytokine immunogens can be administered to the animal in any suitable form and by any suitable route. It is most preferred if the cytokine immunogens are prepared as a sterile, pyrogen-free composition preferably also containing an adjuvant. The cytokine immunogens may be prepared individually or as a mixture. The most suitable route of administration is by injection. Preferably the cytokine immunogen is administered by subcutaneous injection or by intraperitoneal injection but, in any case, slow release of the cytokine immunogen is preferred.

It is preferred if the cytokine immunogens are administered to the animal contemporaneously, for example by administration of a composition containing a mixture of the cytokine immunogens. However, the cytokine immunogens may be administered sequentially, for example by separate administration of compositions containing individual cytokine immunogens.

The number of cytokine immunogens administered to the animal is preferably less than ten; more preferably it is four; more preferably still it is three and most preferably it is two.

The cytokine immunogen may be any cytokine or immunogenic fragment or variant thereof. Immunogen consisting of or comprising a peptide sequence of an exposed epitope of the cytokine are particularly preferred. Preferably, the cytokine is a cytokine which mediates some disease state. Such cytokines include TNFα, IL-1β, IFNγ, and IL-8 which appear to be involved, at least to some extent, in mediating shock and shock-like conditions.

Thus, in a preferred embodiment the cytokines administered to the animal are selected from TNFα, IL-1β, IFNγ, and IL-8. It is particularly preferred if the cytokines administered to the animal are TNFα and IL-1β, or TNFα, IL-1β and IFNγ, or IL-1β and IFNγ, or TNFα and IFNγ. It is preferred if the cytokine immunogen is not IL-6.

Although the cytokine immunogens can be administered to the animal in any ratio which will still give rise to an immune response against all of the cytokine immunogens so administered, it is convenient to administer the cytokine immunogens in equal amounts by weight.

The animals can be any suitable animals including sheep, horse, goat and chicken. Sheep is preferred.

We have unexpectedly found that administering at least two cytokine immunogens to an animal (particularly when administered as a mixture of at least two cytokine immunogens) is not detrimental to antiserum quality or yield. The cytokine-specific antibody concentrations in animals administered at least two cytokine immunogens were found to be virtually the same as those produced when the animals were administered with a single cytokine immunogen. These findings are surprising and unexpected. The total serum immunoglobulin G concentration of immunized adult Welsh Half Bred ewes ranges between 24-28g/l, thus, the 3-4g/l of cytokine-specific antibody achieved in flocks 1 and 2 (see Example 1) represents approximately 15% of the available circulating antibody. It would, therefore, be expected that immunizing with two separate cytokines would yield diminishing returns in specific antibody concentration otherwise the animal would become rapidly immuno-compromised. Cytokine-specific antibody concentrations and titres in serum from the mixed immunogen (polykine) flock were, however, indistinguishable from those in samples taken from sheep receiving a single immunogen and it would seem, therefore, that separate and equivalent antibody populations are stimulated for each cytokine. The average ELISA titre for each cytokine was also similar in all flocks, again suggesting antisera of similar avidity as well as concentration. This approach to the production of a mixture of polyclonal antibodies which bind to at least two cytokines has a number of benefits for commercial antiserum production particularly when using sheep) (i) by employing sheep, which are low cost and which invariably yield a neutralizing antiserum, the many likely combinations of mixed cytokine therapies may be studied with minimum initial outlay and greater certainty of success; and (ii) the smaller numbers of sheep required reduces farming costs and is sympathetic to animal welfare concerns. Similar advantages are to be found when using other animals.

Furthermore, the method allows the production of antiserum with, in the case of using two cytokine immunogens, doubled amounts of cytokine specific antibody in a single preparation. This is a particular benefit when the preparation is used therapeutically as discussed below.

The antibodies produced using the method of the invention may be purified using well known methods. For some applications whole antibodies may be preferred but for other applications (particularly therapeutic applications) it may be preferred to produce Fab or $F(ab)'_2$ fragments.

Methods of producing Fab fragments are described in WO 94/29348, incorporated herein by reference. Conveniently, the Fab fragments are purified by ion-exchange chromatography or by affinity chromatography using a mixture of cytokines (or cytokine immunogens) as affinity ligand. For pharmaceutical use it is preferred that the antibodies have been salt precipitated, digested with papain and the Fc fragment digested and removed by ultrafiltration and the resultant Fab fragments formulated for use.

$F(ab)'_2$ fragments can be produced from intact antibodies using methods known in the art.

A second aspect of the invention provides a mixture of polyclonal antibodies obtainable by the method of the first aspect of the invention.

A third aspect of the invention provides an immunogenic formulation comprising a mixture of cytokine immunogens and an adjuvant. This formulation is suitable for administering the cytokine immunogens in the first aspect of the invention. Suitable adjuvants are well known in the art of antibody production and vaccination. Freund's complete adjuvant, Freund's incomplete adjuvant, $Al(OH)_3$), mineral oil, iscoms and muramyl dipeptide adjuvant are suitable adjuvants.

It is preferred if the cytokine immunogen is an intact, native cytokine or variant, fragment, or fusion thereof.

It is further preferred if the cytokine immunogens are human cytokines.

A particularly preferred immunogenic formulation comprises TNF$\alpha$ and IL-1$\beta$. Suitably the TNF$\alpha$ and IL-1$\beta$ are present in equal amounts.

The mixture of polyclonal antibodies produced by the method of the first aspect of the invention is useful in preventing or ameliorating septic shock and shock-like conditions. Fab fragments of the polyclonal antibodies are particularly useful. Purified antibodies or Fab fragments thereof are conveniently lyophilised for pharmaceutical use.

It is most preferred if the mixture of polyclonal antibodies (or Fab fragments thereof) is used to treat a human patient.

The aforementioned polyclonal antibodies produced by the method of the invention, or a formulation thereof, may be administered by any conventional method including parenteral (eg intravenous or intramuscular) injection. The treatment may consist of a single dose or a plurality of doses over a period of time.

Whilst it is possible for a compound of the invention to be administered alone, it is preferable to present it as a pharmaceutical formulation, together with one or more acceptable carriers. The carrier(s) must be "acceptable" in the sense of being compatible with the compound of the invention and not deleterious to the recipients thereof. Typically, the carriers will be water or saline, or suitable buffers, which will be sterile and pyrogen free.

Formulations suitable for parenteral administration include aqueous and non-aqueous sterile injection solutions which may contain anti-oxidants, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared. Preferred unit dosage formulations are those containing a daily dose or unit, daily subdose or an appropriate fraction thereof, of an active ingredient.

Thus, a fourth aspect of the invention provides a method of preventing or ameliorating septic shock or a shock-like condition in a patient comprising administering to the patient a therapeutically effective amount of the mixture of polyclonal antibodies obtained by the method of the first aspect of the invention.

It is most preferred if the mixture of polyclonal antibodies (or Fab fragments thereof) is used to treat a human patient.

A fifth aspect of the invention provides use of a mixture of polyclonal antibodies obtained by the method of the first aspect of the invention in the manufacture of a medicament for treating shock or a shock-like condition.

A sixth aspect of the invention provides a pharmaceutical composition comprising a mixture of polyclonal antibodies obtained by the method of the first aspect of the invention and a pharmaceutically acceptable carrier.

It is preferred if the antibodies in the formulation are not encapsulated. It is further preferred if the antibodies are not encapsulated in a protein and not encapsulated using an oil emulsion method. It is preferred if the antibodies are free in solution prior to administration to the patient.

A seventh aspect of the invention provides a method of preventing or ameliorating septic shock or a shock like condition in a patient comprising administering to the patient an antibody reactive towards TNFα and an antibody reactive towards IL-1β in a therapeutically effective combination.

It is most preferred if the mixture of polyclonal antibodies is used to treat a human patient.

By "therapeutically effective combinations" we mean a combination which prevents or ameliorates septic shock or a shock-like condition. The skilled person can readily determine whether a combination is therapeutically effective. A therapeutically effective amount is an amount that will substantially reduce the amount of IL-1β and TNFα in the patient's serum. Preferably, the amount is sufficient to reduce serum IL-1β and TNFα to levels that cannot be detected. A therapeutically effect amount is also an amount that can decrease the incidence of mortality during a 28 day period post drug administration; or an amount which is shown by its APACHE II score (Acute Physiology and Chronic Health Evaluation) to be therapeutically effective.

We have found that the administration of an antibody reactive towards TNFα and an antibody reactive towards IL-1β to a patient is unexpectedly beneficial compared to the administration of either an antibody reactive towards TNFα or an antibody reactive towards IL-1β alone.

The antibodies may be administered contemporaneously or sequentially. It is more preferred if they are administered contemporaneously and less preferred if they are administered sequentially. It is further preferred if substantially equal amounts of antibody reactive towards TNFα and antibody reactive towards IL-1β are administered.

It is preferred if one or both antibodies is a polyclonal antibody although monoclonal antibodies may be used, and it is particularly preferred if a Fab fragment of the antibody is used.

The Fab fragments may be generated from substantially pure IgG by methods well known in the art and disclosed in the Examples.

It is further preferred if the IgG Fab fragments are derived from polyclonal antiserum. Polyclonal antiserum (which includes polyclonal IgG) can be produced by immunizing a sheep, goat, horse or other mammal. It is preferable if the mammal is a sheep, and that it is free of scrapie and zoonotic viruses. Methods of making a mixture of Fab fragments reactive towards TNFα and IL-1β and derived from polyclonal sheep IgG are described in the Examples.

A suitable dose of a polyclonal anti-TNFα or anti-IL-1β Fab fragment is between 0.5 mg/kg and 20 mg/kg.

An antibody reactive towards TNFα and an antibody reactive towards IL-1β in a therapeutically effective combination are useful in the treatment of various medical conditions which we call septic shock or a shock like condition. Septic shock may occur following bacterial infection, particularly infection with Gram-negative bacteria, and during septicaemia. By shock we also include trauma following an accident or surgery. Further uses include treatment in conjunction with anti-lymphocyte antibody therapy, as taught in WO 89/08460, and in conjunction with cancer chemotherapy, as taught in EP 355 067.

It is preferred if the said therapeutically effective combination is used to treat patients with SIRS. It is particularly preferred if the therapeutically effective combination is used to treat patients with bacteraemia.

It is known that intravenous infusions of laprine or equine polyclonal antibodies directed against human lymphocytes are very effective in helping to treat acute renal allograft rejection. Such products are still in use. Following clinical trials, Ortho Pharmaceutical Co was granted a licence by the FDA in 1986 for the routine use of a murine monoclonal antibody, OKT3, to help to prevent graft rejection following renal transplant. This product has proved extremely effective in the treatment of acute rejection episodes following kidney, liver and heart transplants.

OKT3 binds specifically to the CD-3 complex found on all mature T lymphocytes. CD-3 is normally involved in antigen recognition and cell stimulation and both are prevented, due to steric hindrance, by the presence of the murine antibodies.

The hybridoma expressing the monoclonal antibody OKT3 is available from the America Type Culture Collection, 12301 Parklawn Drive, Rockville, Maryland 20852 USA under accession number ATCC CRL 8001. It is of the IgG2a isotype and reactive to human helper T cell subset. The hybridoma and antibody are cited in US Patent 4,381,295 incorporated herein by reference. A treatment regime with OKT3 is described in Ortho Study Group (1985) *N. Engl. J. Med.* **313**, 337-342 incorporated herein by reference.

However, patients have a price to pay, in terms of side-effects, for the undoubted benefits which accrue from such anti-T-lymphocyte therapy. Thus during the first infusion of a polyclonal anti-lymphocyte globulin and following the first injection of OKT3, virtually every patient experiences severe signs and symptoms. These include fever in at least 90% of subjects, headaches, nausea and vomiting, diarrhoea, general malaise and extreme fatigue, dyspnoea, myalgia and tachycardia. A few patients develop acute pulmonary oedema. During the second period of therapy side-effects are minimal and, during all subsequent periods, non-existent.

These clinical manifestations are similar to those encountered following infusions of TNF or in septic shock. Infusions of anti-lymphocyte globulin have been shown to cause a marked increase in circulating TNF from undetectable levels to values ranging from 111 to 731 ng/L. This rise in TNF is closely followed by fever (from 38.4 to 40.4°C).

It is an object of the present invention to reduce the shock-like symptoms following OKT3 or anti-lymphocyte globulin treatment.

Thus, in one preferred embodiment a therapeutically effective combination of an antibody reactive towards TNFα and an antibody reactive towards IL-1β is used to treat patients who are receiving the monoclonal antibody OKT3, or functionally equivalent polyclonal antisera, for example in order to reduce acute rejection during kidney transplantation. The said therapeutically effective combination reduces the shock-like side effects of the treatment with OKT3 or functionally equivalent antibodies.

Louse-borne relapsing fever (LBRF), caused by the spirochaete *Borrelia recurrentis,* has been responsible for massive pandemics in Europe, Africa and the Middle East during this century but it is currently restricted to an endemic focus in the highlands of Ethiopia where more than 10,000 cases are reported each year. During epidemics the mortality of the untreated disease may exceed 40%, (even exceeding 50% in some epidemics) but is reduced to less than 5% with antimicrobial agents including penicillin, tetracyclines, chloramphenicol and erythromycin. Clinical cure, can only be achieved by the use of antibiotics which induce a violent and sometimes fatal Jarisch-Herxheimer reaction (JHR). Intravenous tetracyclines cause such a reaction which starts predictably within about an hour of treatment and consists of three phases - rigors, flush and defervescence - of the classical "endotoxin" or "shock" reaction (Warrell *et al* (1970) *Clin. Sci.* **39**, 123-145).

The violent rigors associated with increases in pulse and respiratory rates, blood pressure and temperature. This is followed by a "flush phase" of peaking temperature, vasodilatation, falling mean arterial pressure, and eventually, sweating. Finally there is a "deference" with all variables returning to normal over several hours. Spirochaetes disappear and the leucocyte count falls during the first few hours of this J-HR. During the J-HR, patients may die, either of hyperpyrexia at the peak of the reaction or, more commonly, of myocardial failure with acute pulmonary oedema and shock during the prolonged flush phase. Studies in Addis Ababa over the last 25 years have shown that corticosteroids and antipyretics, such as paracetamol, have little or no effect on the life-threatening cardiorespiratory complications of the J-HR. Meptazinol, an opioid agonist/antagonist, ameliorated some features of the reaction but did not prevent it. The J-HR of LBRF thus resembles a classical "endotoxin" reaction and is a model for other forms of shock and shock-like conditions.

Negussie and his colleagues ((1992) *J. Exp. Med.* **175**, 1203-1207) documented the explosive release of TNFα, followed by IL-6 and IL-8 during this JHR.

Thus, in a further embodiment a therapeutically effective combination of an antibody reactive towards TNFα and an antibody reactive towards IL-1β is used to treat patients with symptoms of septic shock as evidenced by the Jarisch-Herxheimer reaction.

Suitably the JHR is associated with the antibiotic treatment of louse-bornerelapsing fever.

It is well known that JHR, or a similar reaction, is associated with antibiotic treatment of various other parasitic and invading organism diseases including Lyme disease, syphilis, tick-borne relapsing fever, Vincent's angina, rat-bite fever, leptospirosis, yaws, brucellosis and African trypanosomiasis.

Further aspects of the invention provide use of an antibody reactive towards TNFα and an antibody reactive towards IL-1β, in combination, in the manufacture of a medicament for preventing or ameliorating septic shock or a shock-like condition in a patient; use of an antibody reactive towards TNFα in the manufacture of a medicament for treating a patient who has been, is being or will be administered an antibody reactive towards IL-1β for preventing or ameliorating septic shock or a shock-like condition; use of an antibody reactive towards IL-1β in the manufacture of a medicament for treating a patient who has been, is being or will be administered an antibody reactive towards TNFα for preventing or ameliorating septic shock or a shock-like condition; and a pharmaceutical formulation comprising an antibody reactive towards TNFα and an antibody reactive towards IL-1β and a pharmaceutically acceptable carrier.

It is preferred if the antibodies in the formulation or medicament are not encapsulated. It is further preferred if the antibodies are not encapsulated in a protein and not encapsulated using an oil emulsion method. It is preferred if the antibodies are free in solution prior to administration to the patient.

Formulations can be prepared and administered essentially as described for the third aspect of the invention.

The invention will now be described in more detail with reference to the following Examples and Figures wherein:

Figure 1 shows a typical ELISA dilution curve which plots $OD_{492}$ against antiserum dilution.

Figure 2 shows a typical profile of a small scale affinity purification.

Figure 3(a) shows the variation in monthly average of the TNFα-specific antibody concentration in flocks 1 and 3.

Figure 3(b) shows the variation in monthly average of the IL-1β-specific antibody concentration in flocks 2 and 3.

Figure 4(a) shows the variation in the monthly average ELISA titre of TNFα-immune samples in flocks 1 and 3.

Figure 4(b) shows the variation in the monthly average ELISA titre of IL-1β-immune samples in flocks 2 and 3.

Figure 5 shows the effect of cytokine antibodies on the vascular hyporeactivity to noradrenaline (NA) induced by lipoteichoic acid (LTA).

Figure 6 shows the effect of cytokine antibodies on the hypotension induced by LTA. MAP is mean arterial pressure.

Figure 7 shows that cytokine antibodies attenuate the enhanced-inducible nitric oxide synthase (iNOS) activity in the lung homogenates.

**Example 1: Production of polyclonal antibodies directed at TNFα and IL-1β**

**Methods**

**Primary Antiserum Production**

Groups of up to 150 Welsh Half Bred ewes were immunized with either:

(i) recombinant human TNFα (R and D Systems Ltd, Abingdon, UK; Flock 1)
(ii) recombinant human IL-1β (PeproTech Inc, Rocky Hill, NJ, USA; Flock 2) or
(iii) a 1:1 mixture (by weight) of the above (Flock 3; polykine flock).

For the purposes of these studies, sheep in all flocks received 50µg of cytokine or cytokine mixture. Our previous unpublished studies had, however, shown that varying the immunizing amounts between 40 and 160µg did not affect the antibody response. For primary immunization, immunogens were emulsified in Freund's complete adjuvant but Freund's incomplete adjuvant was used for subsequent immunizations.

Sheep were injected on a monthly basis at six separate subcutaneous sites. Sheep were bled, two weeks after each immunization, taking 10ml of blood per kg of body weight. After clotting, the immune serum was aspirated and stored at -20°C until required.

**Antiserum Assessment**

Antisera were assessed for their titre and specific antibody concentration.

**ELISA Titre Determinations**

96 well plastic microtitre plates were coated, at a concentration of 0.2µg per well, with either TNFα or IL-1β dissolved in sodium bicarbonate buffer (pH 9.6, 0.1M). Plates were incubated with the immunogen for 2h at room temperature. Coated plates were then washed (5 x 100µl) with PBS/Tween (0.02%) and then blocked by incubation with the same solution for 2h at 37°C. Plates were stored at -20°C until required.

Test antisera were diluted in PBS/Tween and applied (100µl), in duplicate, to coated plates. Doubling dilutions of antiserum were then made in the plate and the mixture allowed to reach equilibrium for 1h at 37°C. After washing as before, bound specific antibody was detected by addition of donkey anti-sheep IgG (100µl, 1:500 dilution in PBS/Tween) to which was conjugated horseradish peroxidase. Plates were again washed and then developed by the addition (100µl) of orthophenylene diamine (100mg sodium citrate buffer; 70mM, pH 5.0) activated by hydrogen peroxide (100µl).

Plates were allowed to develop for 15 min in the dark and the reaction then terminated by the addition of sulphuric acid (3M, 100µl). Sample absorbance was read at 492 nm and the titre determined at 50% of the background corrected maximum reading (Figure 1).

**Small Scale Affinity Purification**

The specific antibody concentration in antiserum samples was determined using small (1-2.0g) CNBr-Sepharose-IL-1β or CNBr-Sepharose-TNFα affinity columns. Ligand (either IL-1β or TNFα) was conjugated to the matrix at a concentration of 2mg per gram of dry affinity gel following the manufacturer's instructions. Ligand was dissolved in sodium bicarbonate buffer (pH 8.3, 0.1M containing sodium chloride, 0.5M) and added to the dry gel which had previously been washed with hydrochloric acid (1mM). Conjugation was allowed to proceed for 16h at 4°C and the unbound protein then run off. The remaining active groups on the matrix were blocked by incubation with ethanolamine (pH 8.0, 1.0M containing sodium chloride, 0.5M) for 2h at room temperature. The gel was then washed with buffers of alternating pH (bicarbonate coupling buffer or sodium acetate, pH 4.0, 0.1M containing sodium chloride 0.5M).

Test samples, diluted in saline (sodium chloride, 0.15M) were applied to the column to form a 50% slurry and then allowed to mix with the matrix for 16h at 4°C. The unbound material was then run off and the column washed with 5 column volumes of saline. Bound specific antibody was eluted using glycine buffer (0.1M, pH 2.5), and the concentration determined by optical density measurements at 280nm (Figure 2).

Columns were calibrated by loading increasing amounts of serum until saturation was achieved. For routine assessment, columns were loaded at 35-50% of their total capacity.

**Results**

**Specific Antibody Concentrations**

Figure 3 represents the cytokine-specific antibody concentration of pooled monthly serum samples from the three test flocks for a six month period. In general, TNFα-specific antibody levels in flocks 1 and 3 (Figure 3a) rose and fell in parallel over the test period, their average concentrations differing by only 0.5% during this time (3.39 cf 3.37g/1 for flocks 1 and 3 respectively). IL-1β-specific antibody levels in flocks 2 and 3 (Figure 3b) described a similar pattern and, again average specific antibody concentrations differed by less than 1 %. These differences were within the errors associated with the assay (5%).

**ELISA Titres**

Individual serum titres were determined for each sheep. All responded well to immunization (not shown). Figure 4 shows the averaged monthly ELISA titres from these sheep for the 6 month period. Titres, in all flocks, were exceptionally high ranging from 1:900,000 to 1:1,600,000 and, while not exhibiting such close correlation as small scale affinity data, the cytokine-specific titres of flock 3, in general, paralleled those achieved in flocks dedicated to a single cytokine. Thus, the average difference in ELISA titre between single and mixed cytokine flocks during this period was 14% and 3% for IL-1β and TNFα respectively. The average coefficient of variation for the ELISA assay was 8%.

Values for both small scale affinity purification and ELISA reached plateau levels within three months of primary immunization.

**Discussion**

These results show that immunising sheep with a mixture of at least two cytokines is not detrimental to antiserum quality and yield. Thus cytokine-specific antibody concentrations in the mixed immunogen flock were virtually the same as those produced when sheep were immunized with only one cytokine and ELISA titres (reflecting both the amount and avidity of the antibodies; Butler *et al* (1978) *J. Immunochem.* **15,** 131) for flock 3 were equal to those achieved in flocks 1 and 2. These findings are surprising and unexpected.

**Example 2: *In vivo* effects of administration of a mixture of anti -TNFα and anti-IL-1β antibodies**

The mixture of anti-TNFα and anti-IL-1β antibodies was made according to the method in Example 1.

Primary *In Vitro* Serum Analysis of the mixture used (polykine antibody) for administration is:

| Flock | ELISA Titre x 1000 | | SSAP (g/L) | |
|---|---|---|---|---|
| | vs TNFα | vs IL1-β | vs TNFα | vs IL1-β |
| αIL1-β/TNFα | 644 | 574 | 3.2 | 3.7 |

SSAP is a small-scale affinity purification and is described in Example 3.

(a) A murine Gram positive sepsis model has been developed where doses of lipoteichoic acid (LTA) are used to induce hypotension and, ultimately, organ failure. Studies (whose results are shown in Figures 5 to 7) have shown that a mixture of anti-TNFα antibody and anti-IL-1β antibody is capable of reversing the LTA induced hypotension at a dose of 3 mg/kg. (This is the dose of total antibodies containing an approximately equal percentage of antibodies specific to TNFα and IL-1β.) Antibodies to each of the individual cytokines are less able to reverse this effect and, at a dose of 3 mg/kg the reversal is not significant (Figure 6).

(b) In addition, a mixture of anti-TNFα antibody and anti-IL-1β antibody is shown to be more effective at reversing the LTA induced hyporeactivity to noradrenaline (NA) in this model than either of the antibodies to each of the individual cytokines (Figure 5).

NB: Blood pressure is maintained by sympathetic release of noradrenaline which has a pressor effect. Nitric oxide, released by the endothelium in response to an LTA challenge, can overcome the effects of noradrenaline making the tissues hyporeactive.

c) The iNOS (inducible nitric oxide synthase) activity of lung homogenates from animals given a bolus injection of LTA may be measured by monitoring conversion of L-arginine to L-citrulline:

$$\begin{array}{c}\text{LTA}\\ +\\ \text{iNOS}\\ {}^{[3H]}\text{L-Arg} \Rightarrow {}^{[3H]}\text{L-Citrulline} + \text{NO}\end{array}$$

L-arginine and L-citrulline may be separated by ion-exchange.

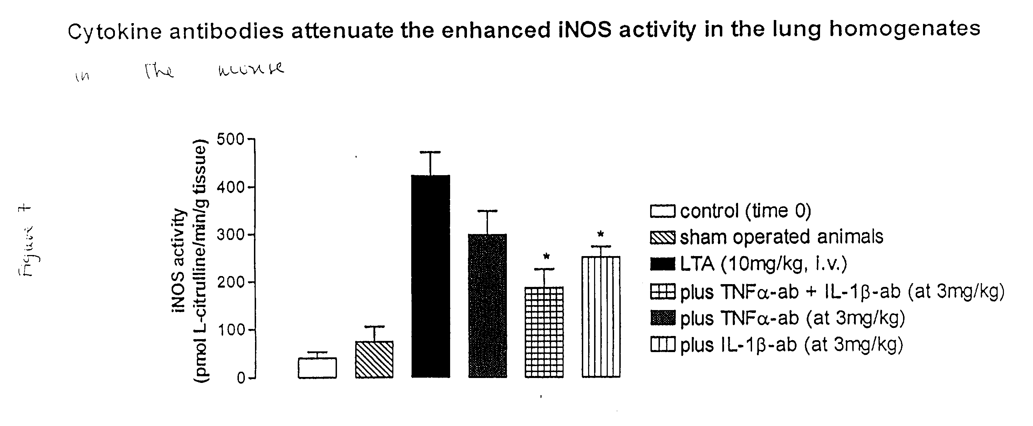

In experiments to monitor iNOS activity by measuring L-citrulline formation, a mixture of anti-TNFα antibody and anti-IL-1β antibody was, again, more effective than either individual TNFα or IL-1β antibodies in attenuating the effects of LTA (Figure 7).

All these experiments suggest that the administration of an anti-TNFα antibody and an anti-IL-1β antibody is more effective than either antibody alone in reversing the effects of LTA in a murine Gram-positive sepsis model.

**Example 3: Small scale affinity purification of antisera for specific antibody concentration determinations**

**Preparation of the Affinity Column**

**1.0 Materials**

**1.1** Chromatography Column (BIORAD, Econocolumn 1.0 x 10 cm, Catalogue No 737-1010)
**1.2** Buchner Flask
**1.3** Water Pump
**1.4** End-Over-End Mixer
**1.5** Clamp Stand
**1.6** Sintered Glass Funnel
**1.7** LKB Biochem Spectrophotometer

**2.0 Chemicals**

**2.1** CNBr-Activated Sepharose 4B (Coupling Gel) (PHARMACIA, Product No 17-0430-01)
**2.2** Ethanolamine (BDH, "Analar", Product No 10325)
**2.3** Sodium Chloride (BDH, "Analar", Product No 10241)
**2.4** Sodium Acetate (Anhydrous) (BDH, "Analar", Product No 10236)
**2.5** Sodium Hydrogen Carbonate (BDH, "Analar", Product No 10247)
**2.6** Hydrochloric Acid (BDH, "Analar", Product No 10125)
**2.7** Thimerosal (SIGMA, Product No T 5125)
**2.8** Cytokine (Lyophilised)

**3.0 Buffers & Reagents**

| | | | |
|---|---|---|---|
| **3.1** | 1 mM HCl | | |
| **3.2** | Coupling Buffer: | Sodium Hydrogen Carbonate | 0.1M |
| | | Sodium Chloride | 0.5M |
| | | pH 8.3 | |
| **3.3** | Blocking Buffer: | Ethanolamine | 1.0M |
| | | pH 8.0 | |

(continued)

| | | | |
|---|---|---|---|
| **3.4** | Acetate Buffer: | Sodium Acetate<br>Sodium Chloride<br>pH 4.0 | 0.1M<br>0.5M |
| **3.5** | Saline (0.9%): | Sodium Chloride | 0.15M |
| **3.6** | Saline (0.9%) +:<br>Thimerosal (0.01%) | Sodium Chloride<br>Thimerosal | 0.15M<br>0.25mM |

**4.0 Procedure**

**4.1** 5 mg of the ligand/protein to be coupled was weighed out and dissolved in 6 mls of the coupling buffer. OD at 280 nm was measured and the ligand/protein was placed in the chromatography column.

**4.2** 1 g of the CNBr-Activated Sepharose 4B (= 3.5 ml swollen gel) was weighed out. It was re-swollen and washed on a sintered glass filter using the 1 mM HCl (200 mls/g Gel).

**4.3** The swollen gel was scooped out quickly and placed into the chromatography column containing the ligand/protein solution. The solution was allowed to mix either overnight at 4°C or at RTP for 2 hrs on an end-over-end mixer. DO NOT USE A MAGNETIC STIRRER.

**4.4** The eluant which contains unbound ligand/protein was collected. The OD at 280 nm was measured to assess the percentage of ligand/protein bound to the coupling gel.

**4.5** 6 mls of the blocking buffer (to block any remaining active groups) was added to the column containing the gel and allowed to mix overnight at 4°C or for 2 hrs at room temperature.

**4.6** The blocking agent was eluted, and any excess adsorbed protein and blocking buffer was washed away by washing the column through with coupling buffer followed by acetate buffer followed by coupling buffer. This was repeated once or twice.

**4.7** The column was washed through with saline. If it is not to be used immediately, store in Saline + thimerosal. (It contains an antibacterial agent).

**Small-Scale Affinity Purification**

**1.0 Materials**

**1.1** End-Over-End Mixer
**1.2** Clamp Stand
**1.3** LKB Biochem Spectrophotometer
**1.4** 3 ml Plastic Test Tubes
**1.5** Dialysis Tubing
**1.6** Cytokine Sample

**2.0 Chemicals**

**2.1** Glycine (BDH, "Analar", Product No 10119)

**3.0 Buffers and Reagents**

| | | |
|---|---|---|
| **3.1** | Eluting Agent: | Glycine 0.1M pH 2.5<br>Made up in distilled water. |
| **3.2** | Saline (0.9%): | Sodium Chloride 0.15M<br>Made up in distilled water. |

### 4.0 Procedure

**Initial Calibration of the Column:**

**4.1** If stored in Saline + Thimerosal, the column was washed with at least 150 ml of saline to remove all traces of Thimerosal from the gel. 0.05 ml of serum under test was then mixed with 6 mls of saline and the resultant solution applied to the affinity column. All liquid was run from the column and the end cap fitted before addition of the serum/saline solution. The column was then rotated end over end for 2 h at room temperature or overnight at 4°C.

**4.2** The unbound serum was then run off the column and collected. The column was then washed with at least 150 ml of saline and the OD280 nm of the eluant then determined. If readings had returned to 0 (+/- 0.05) the column was ready for elution.

**4.3** The bound material was eluted off with the eluting buffer by collecting 1 ml fractions. The OD at 280 nm was measured to obtain an elution profile of the bound material and the fractions collected until the baseline reading fell back down to 0.0.

**4.4** The column was washed through with saline (150 mls or until the OD at 280 nm reads about 0.0). The column was stored at 4°C in Saline + Thimerosal.

**4.5** The fractions were pooled together and dialysed overnight at 4°C against saline. After dialysis, the volume of the dialysate and the OD at 280 nm were measured and the figures used to calculate the concentration of specific antibodies in the original antisera.

**4.6** This procedure was repeated using increasing amounts of antisera and a graph of applied volume against eluted antibody concentration constructed. From this graph, which should be described by a parabola, the amount of antisera required to produce 80% of the saturation value was determined and this concentration of antisera used for all future samples.

### Example 4: Treatment of patients with louse-borne relapsing fever with polyclonal anti-TNFα and anti-IL-1β Fab fragments

#### Treatment

Freeze dried Fab (ion-exchange purified anti-TNFα/anti-IL-1β) was dissolved in sterile water and then added to saline bags. Doses were 0.5-20 mg/kg of the anti-cytokine antibody. Typical doses of anti-TNFαFab were 80 mg, 400 mg and 1000 mg. In patients whose baseline rectal temperature had not fluctuated by more than 0.5°C over a 30 minute period, 100 ml bags of saline containing FAb was infused intravenously over 30 minutes. After this infusion had been completed, 250 mg tetracycline was infused intravenously over 10 minutes into the opposite arm. This has proved a totally adequate treatment for LBRF in Ethiopia.

#### Investigations

Under some circumstances it was useful to carry out investigations. Into the patient a Teflon cannula was placed in an antecubital vein, fitted with a three way tap and kept patent with heparinised saline (4 drops of heparin 5000 u/ml in 40 ml saline. A baseline blood sample was drawn for immediate measurement of microhaemotocrit and total WBC count. Plasma was aliquoted and stored for full biochemical screening. Immediate biochemical profiles was performed at local laboratories, as required. Urine was stix-tested.

After being shaved, deloused, weighed and measured, patients lay down comfortably in bed. A rectal electronic thermometer probe was inserted. Blood pressure, pulse and respiratory rates were recorded at intervals.

#### Assessment

Under some circumstances it was useful to carry out various assessments.

Measurements of clinical severity (changes in blood pressure, pulse and respiratory rates, temperature), occurrence of a period of rigors and laboratory measures such as concentrations of TNFα, IL-1β, IL-6, and IL-8 carried out. To this end, rectal temperature, blood pressure, pulse rate, respiratory rate and symptoms (such as shivering) were recorded at 15 minute intervals at the following times (0 = tetracycline treatment): -60, -45, -30, -15, 0, +15, +30, +45, +60, +75, +90, +105, +120, +135, +150, +165, +180 min, 4, +4.5, +5, +5.5, +6, +6.5, +7, +7.5, +8, +8.5, +9, +9.5, +10, +10.5, +11, +11.5, +12 hr. Ten millilitre samples for cytokine assays (TNFα, IL-1β, IL-6, IL-8) were taken at -60, -30, 0, +30, +60, +120, +180, +240 min. Spirochaete counts were performed at -60, 0, +240 min, +24 hr. The maximum total volume of blood sampled from a patient was 150 ml. Patients were allowed to eat and drink throughout.

Normally a definite J-HR is expected 40-105 (mean 60) minutes after intravenous tetracycline treatment. Minimum blood pressure are likely to be recorded in the saline controls during the flush phase 2-9 hours after the rigors.

**Handling of blood samples for cytokine and biochemical assays**

Under some circumstances it was useful to handle blood samples for cytokine and biochemical assays. Ten millilitre of blood was placed in endotoxin-free lithium heparin tubes. Plasma was separated immediately by centrifugation (100 g for 5 mins) and frozen at -20°C. Assays of TNFα, IL-1β, IL-6 and IL-8 were carried out. Levels of ovine Fab were determined on all the samples and standard biochemical electrolyte assays were performed.

**Example 5: Treatment of patients with SIRS**

(a) 1 mg/kg of each of ion-exchange purified anti-TNFα Fab and anti-IL-1β Fab, in combination, are administered to a human patient with systemic inflammatory response syndrome.

(b) 10 mg/kg of each of ion-exchange purified anti-TNFα Fab and anti-IL-1β Fab, in combination, are administered to a human patient with systemic inflammatory response syndrome.

**Claims**

**1.** A method of producing a mixture of polyclonal antibodies, the mixture including polyclonal antibodies which bind to at least two cytokines, comprising the steps of:

(1) administering to an animal a sufficient amount of each of a cytokine immunogen to provoke an immune response to each of the cytokine immunogens;
(2) allowing an immune response to develop to each of the cytokine immunogens; and
(3) removing blood from the animal.

**2.** A method according to Claim 1 wherein the said cytokine immunogens are administered contemporaneously.

**3.** A method according to Claim 1 or 2 wherein the number of cytokine immunogens administered is two.

**4.** A method according to any one of Claims 1 to 3 wherein the cytokines are selected from TNFα, IL-1β, IFNγ and IL-8.

**5.** A method according to Claim 3 wherein the two cytokine immunogens are TNFα and IL-1β.

**6.** A method according to any one of the preceding claims wherein an equal amount of each cytokine immunogen is administered to the animal.

**7.** A method according to any one of the preceding claims wherein the animal is a sheep.

**8.** A method according to any one of the preceding claims wherein the total amount of cytokine immunogen administered to the animal in a single administration is between 40 μg and 200 μg per animal.

**9.** A mixture of polyclonal antibodies obtainable by the method of any one of the preceding claims.

**10.** An immunogenic formulation comprising a mixture of cytokine immunogens and an adjuvant.

**11.** An immunogenic formulation according to Claim 10 comprising TNFα and IL-1β.

**12.** A method of preventing or ameliorating septic shock or a shock-like condition in a patient comprising administering to the patient a therapeutically effective amount of the mixture of polyclonal antibodies obtained by the method of any one of Claims 1 to 8.

13. Use of a mixture of polyclonal antibodies obtained by the method of any one of Claims 1 to 8 in the manufacture of a medicament for treating shock or a shock-like condition.

14. A pharmaceutical composition comprising a mixture of polyclonal antibodies obtained by the method of any one of Claims 1 to 8 and a pharmaceutically acceptable carrier.

15. A method of preventing or ameliorating septic shock or a shock like condition in a patient comprising administering to the patient an antibody reactive towards TNFα and an antibody reactive towards IL-1β in a therapeutically effective combination.

16. A method according to Claim 12 or 15 wherein the patient is a human.

17. A method according to Claim 15 wherein the antibody reactive towards TNFα and the antibody reactive towards IL-1β are administered contemporaneously.

18. A method according to Claim 15 to 17 wherein substantially equal amounts of the antibody reactive towards TNFα and the antibody reactive towards IL-1β are administered.

19. A method according to any one of Claims 15 to 18 wherein at least one antibody is a polyclonal antibody.

20. A method according to any one of Claims 15 to 19 wherein at least one antibody is an IgG.

21. A method according to any one of Claims 15 to 20 wherein at least one antibody is a Fab fragment.

22. A method according to any one of Claims 12 or 15 to 21 wherein the shock-like condition is caused by the administration of the monoclonal antibody OKT3 or a functionally equivalent antibody.

23. A method according to any one of Claims 12 or 15 to 21 wherein the shock-like condition is caused by the Jarisch-Herxheimer reaction.

24. Use of an antibody reactive towards TNFα and an antibody reactive towards IL-1β, in combination, in the manufacture of a medicament for preventing or ameliorating septic shock or a shock-like condition in a patient.

25. Use of an antibody reactive towards TNFα in the manufacture of a medicament for treating a patient who has been, is being or will be administered an antibody reactive towards IL-1β for preventing or ameliorating septic shock or a shock-like condition.

26. Use of an antibody reactive towards IL-1β in the manufacture of a medicament for treating a patient who has been, is being or will be administered an antibody reactive towards TNFα for preventing or ameliorating septic shock or a shock-like condition.

27. A pharmaceutical formulation comprising an antibody reactive towards TNFα and an antibody reactive towards IL-1β and a pharmaceutically acceptable carrier.

# Figure 1
## Typical ELISA Dilution Curve

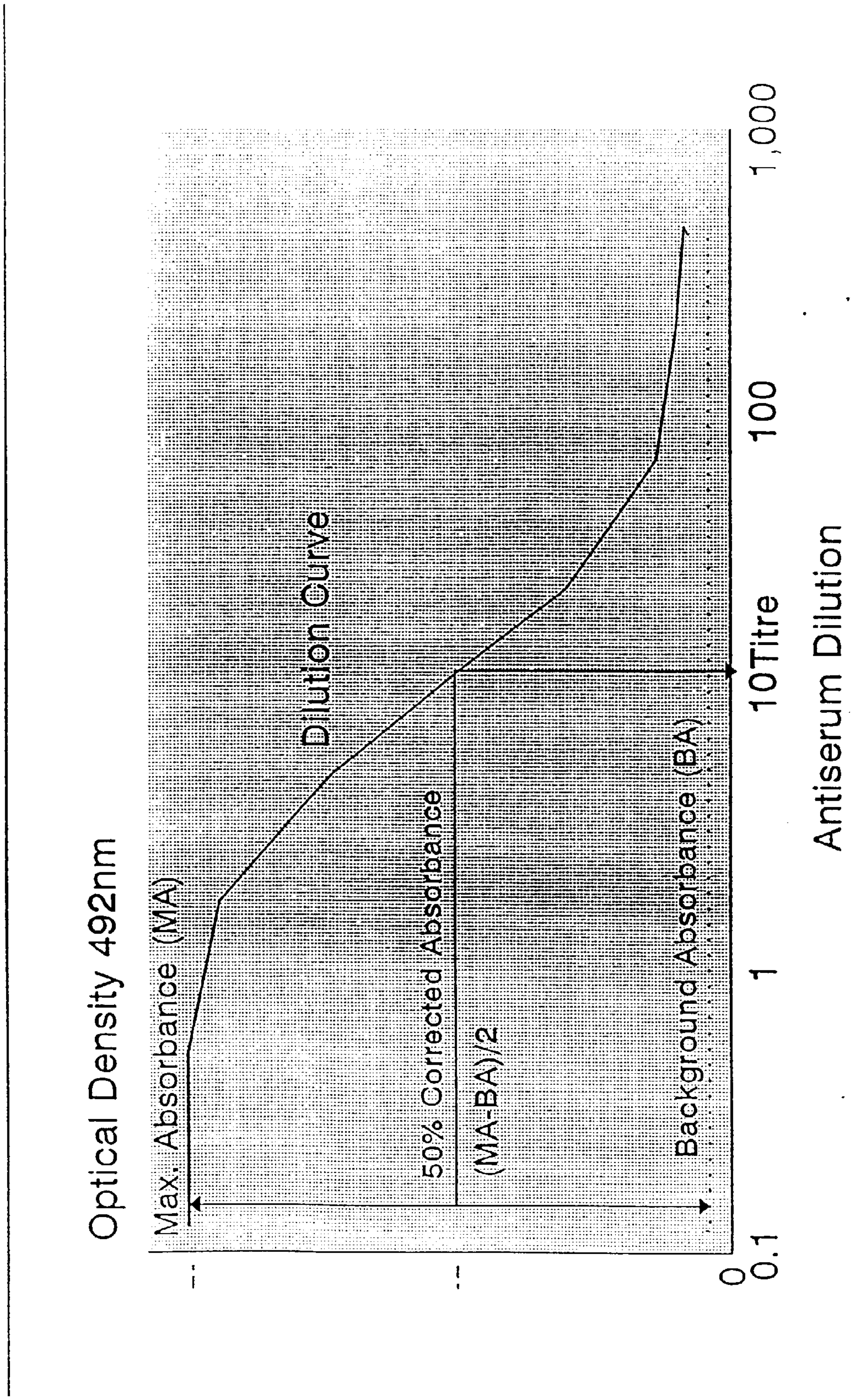


Titre calculated at 50% of the background corrected absorbance

# Figure 2

## Small Scale Affinity Purification: Typical Profile

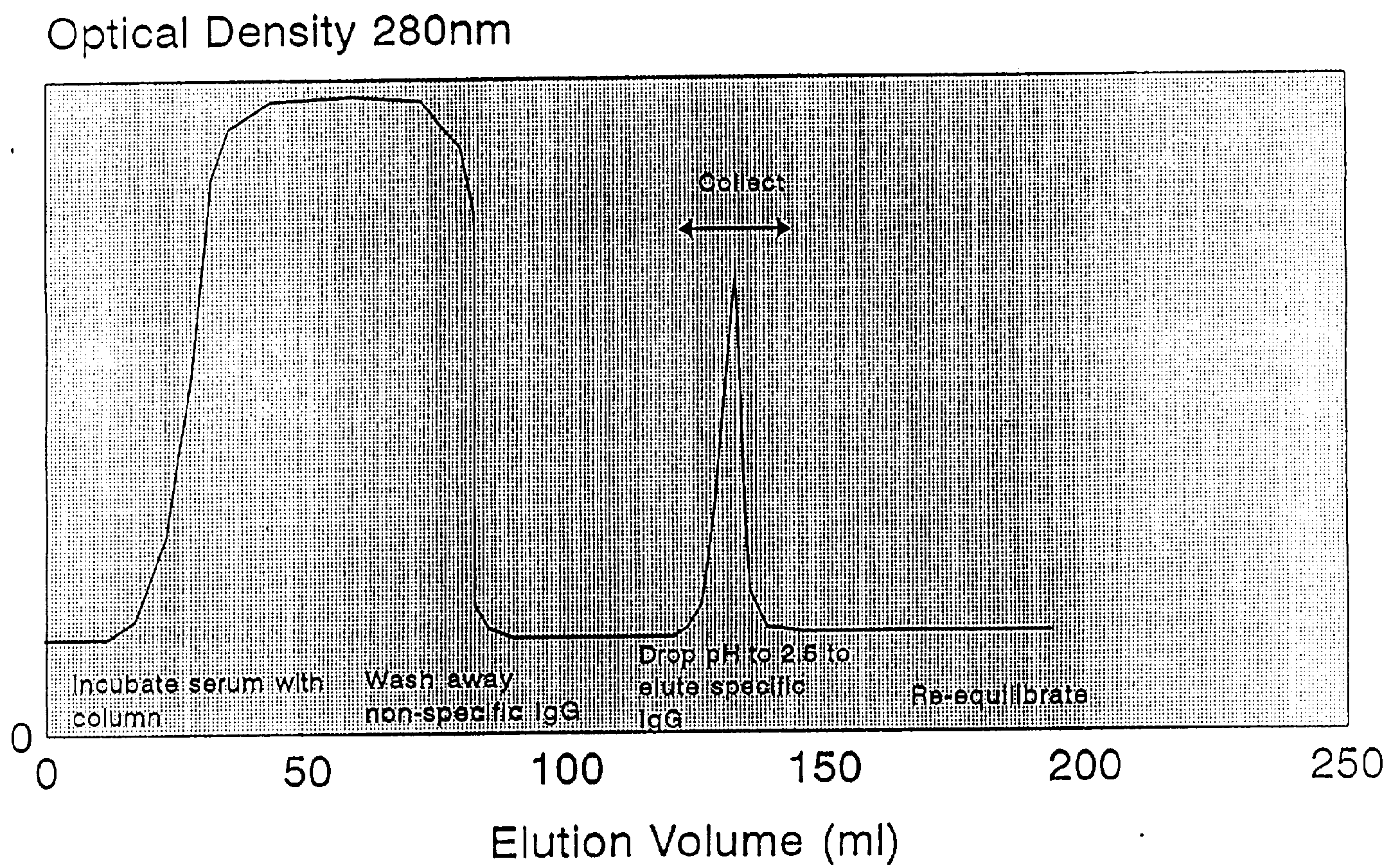


CNBr-Sepharose Column (2.0g)

Figure 3(a)
Variation in Monthly Average TNF Alpha-Specific Antibody Concentration

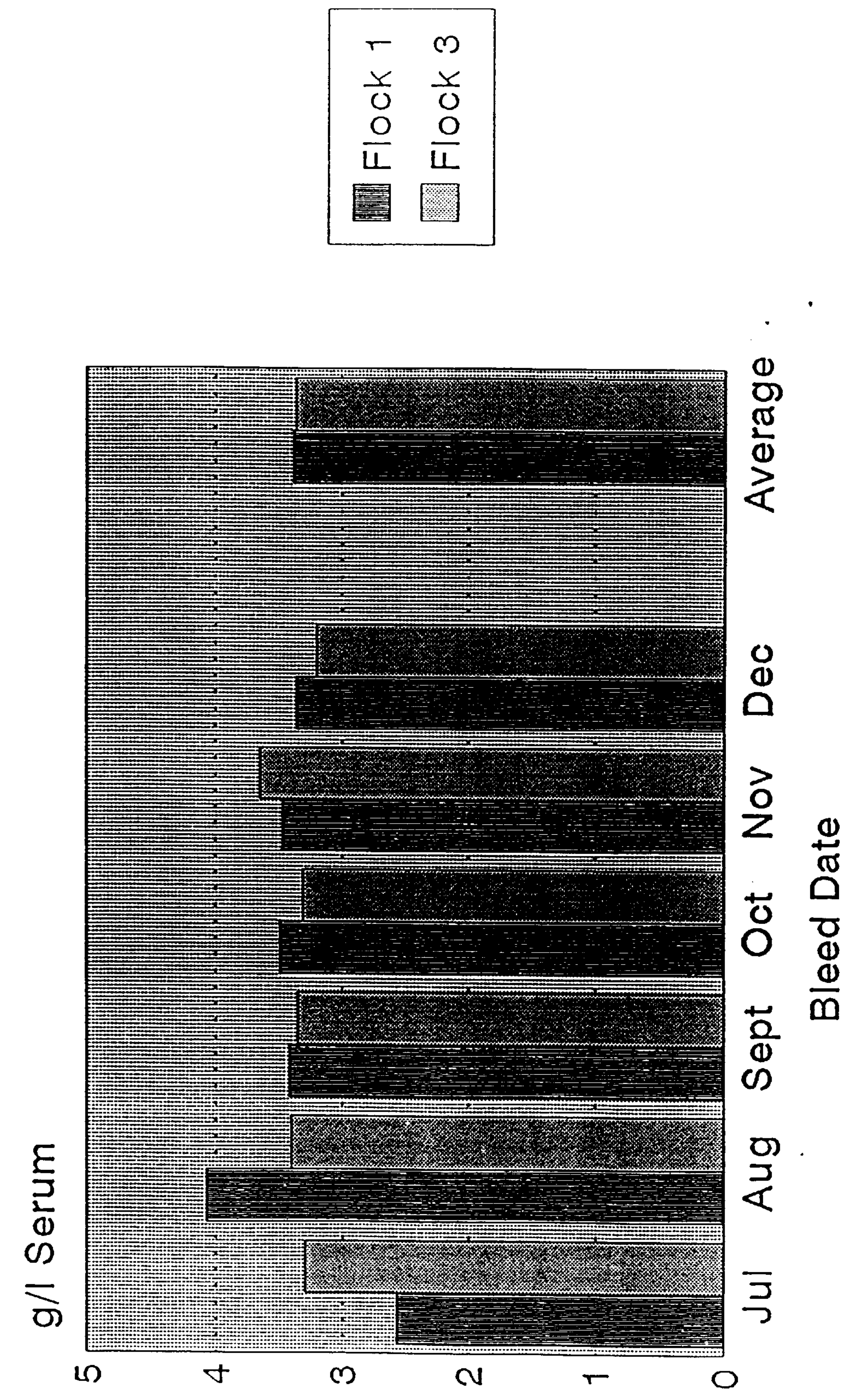

# Figure 3(b)

Variation in Monthly Average IL-1 Beta-Specific Antibody Concentration

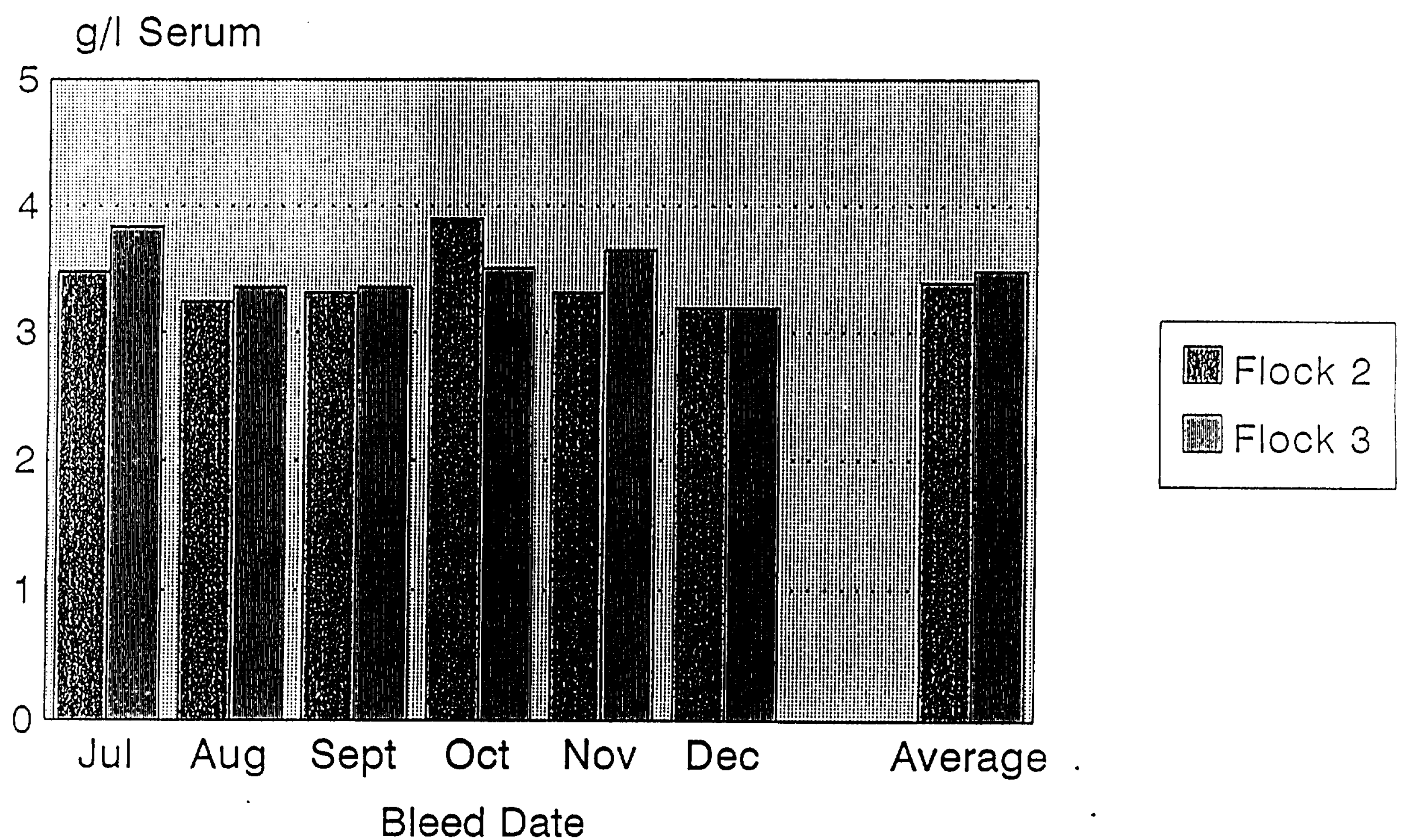

Figure 4(a)

Variation in the Monthly Average ELISA titre of TNF alpha-Immune Samples

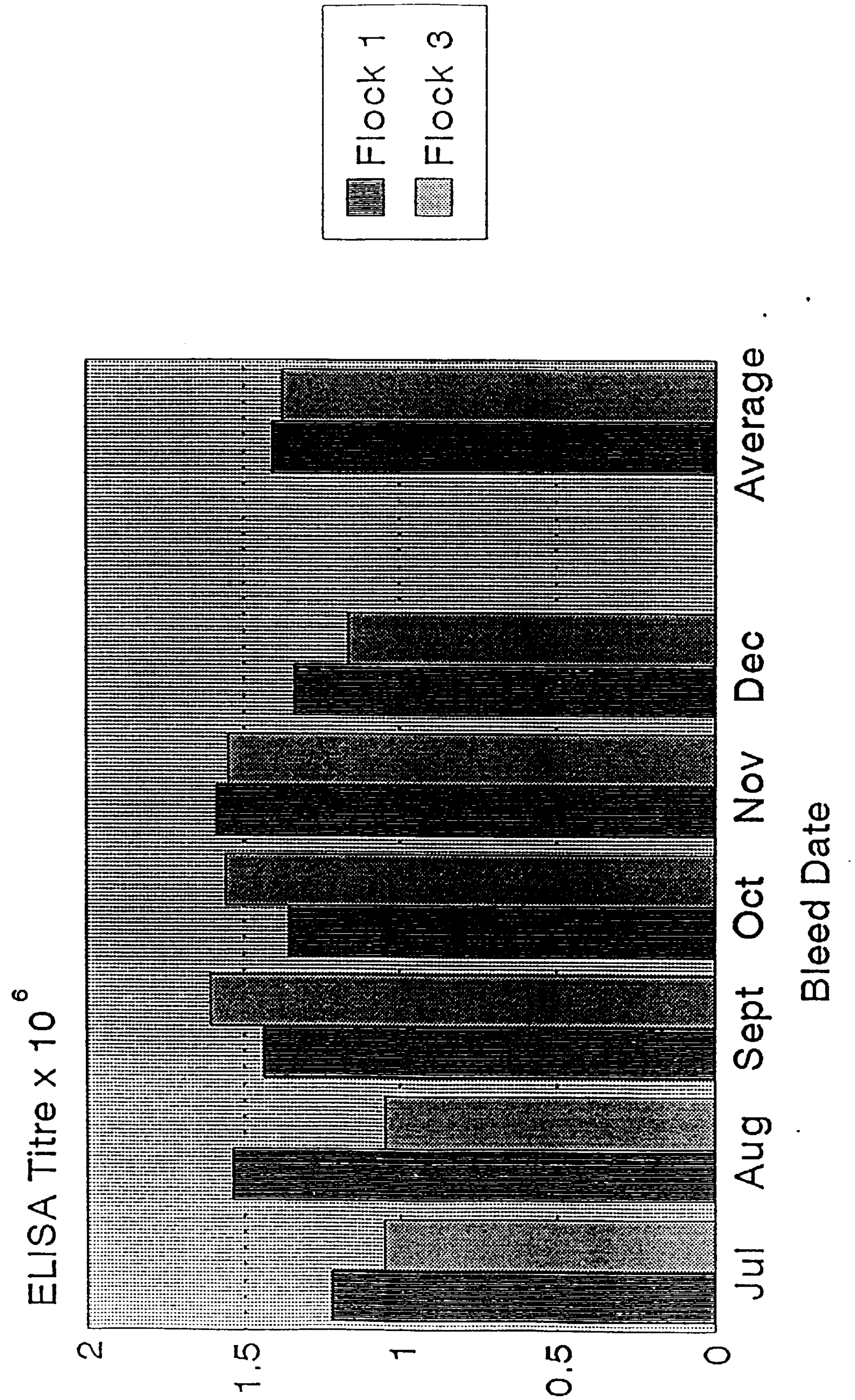

Figure 4(b)
Variation in the Average Monthly ELISA Titre
of IL-1 Beta-Immune Samples

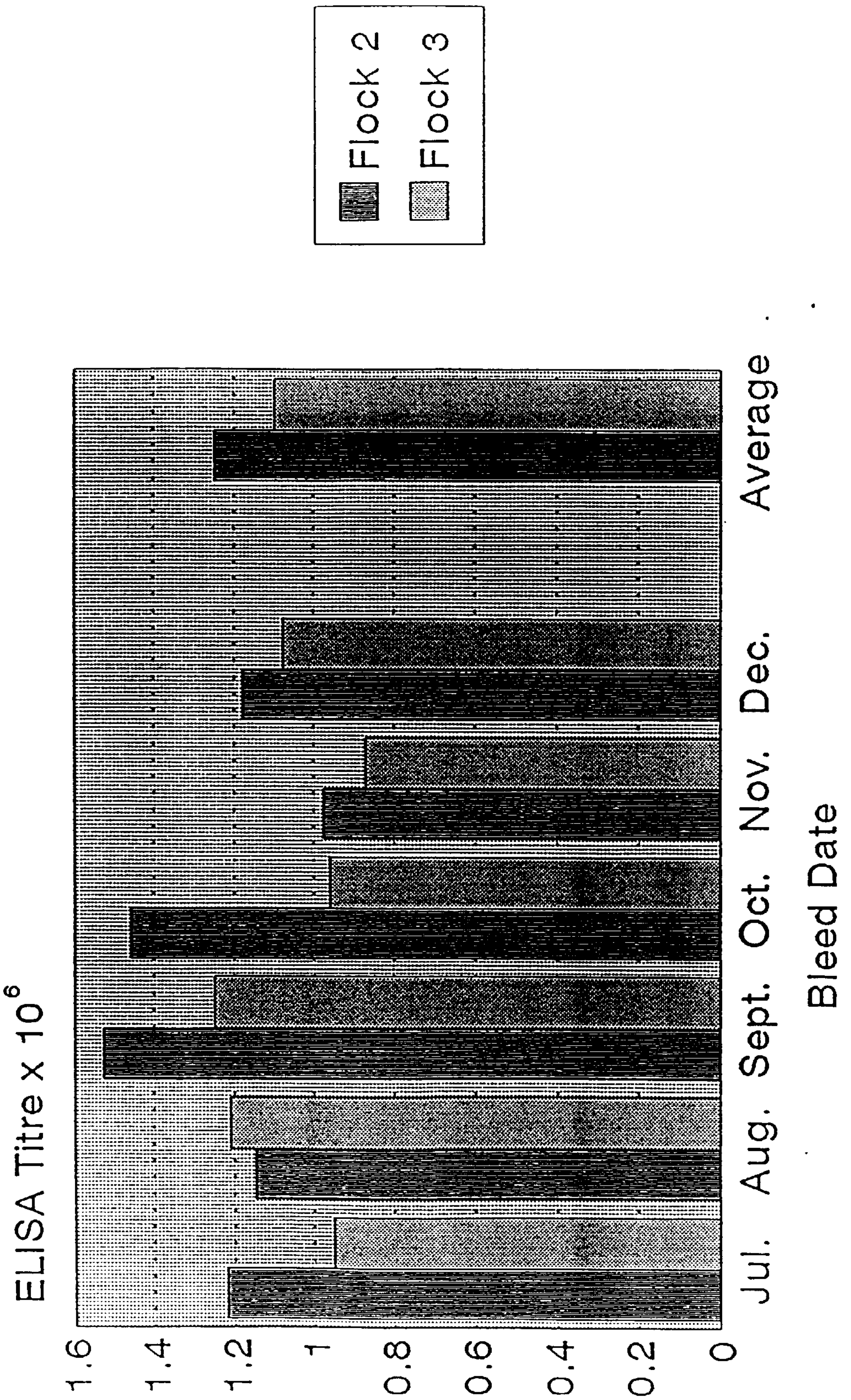

Figure 5

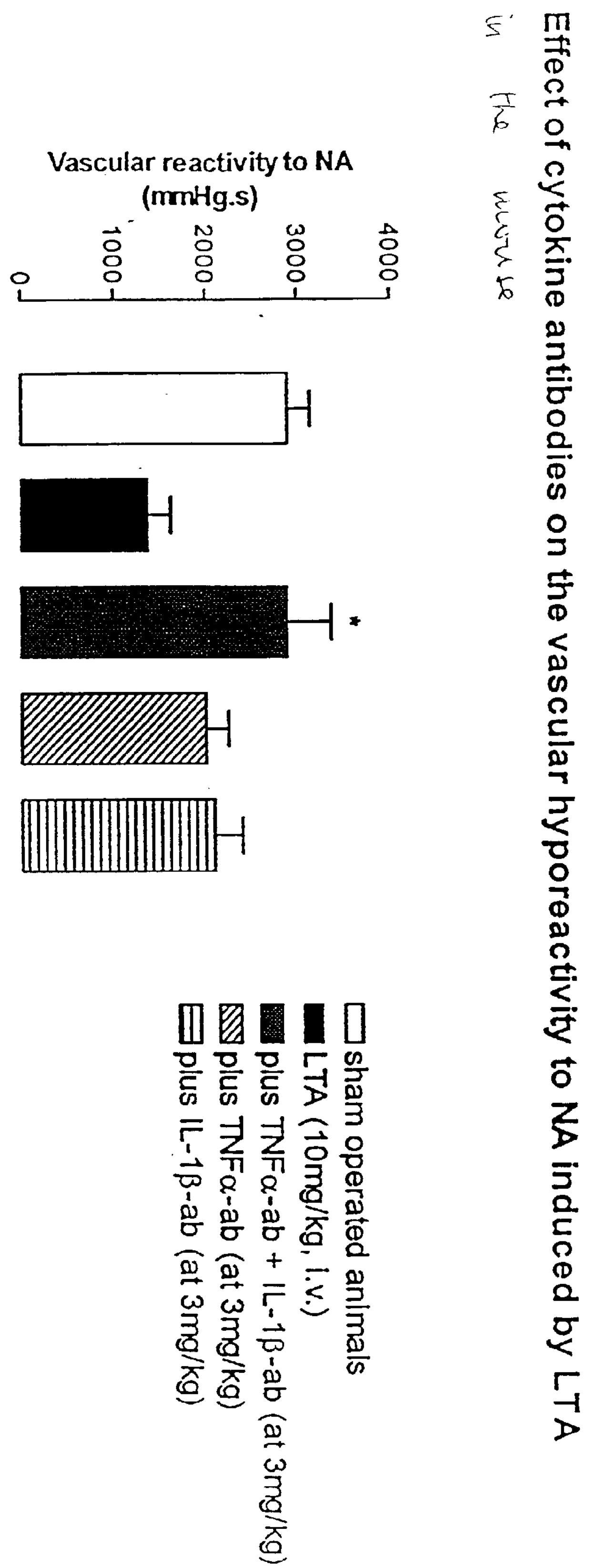
Effect of cytokine antibodies on the vascular hyporeactivity to NA induced by LTA
Vascular reactivity to NA
(mmHg.s)
0
1000
2000
3000
4000
*
sham operated animals
LTA (10mg/kg, i.v.)
plus TNFα-ab + IL-1β-ab (at 3mg/kg)
plus TNFα-ab (at 3mg/kg)
plus IL-1β-ab (at 3mg/kg)

Figure 6

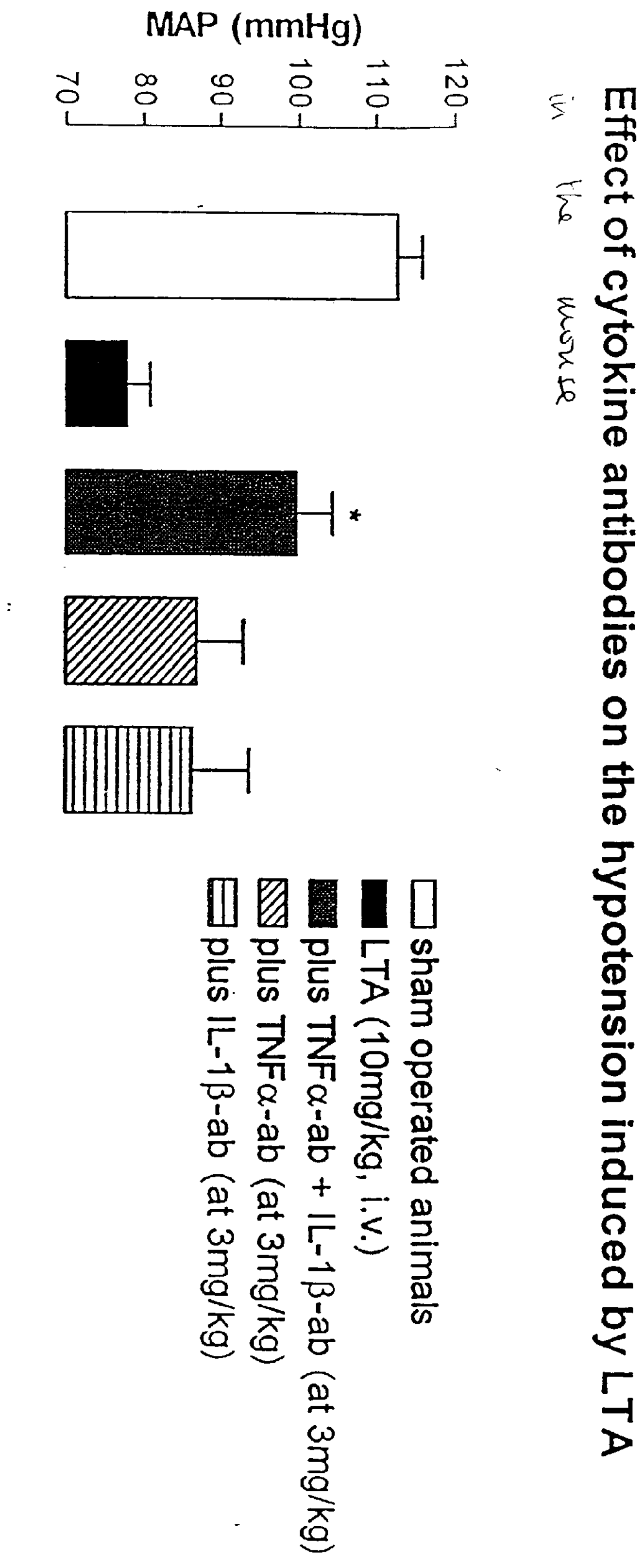
Effect of cytokine antibodies on the hypotension induced by LTA
in the mouse
MAP (mmHg)
70
80
90
100
110
120
*
sham operated animals
LTA (10mg/kg, i.v.)
plus TNFα-ab + IL-1β-ab (at 3mg/kg)
plus TNFα-ab (at 3mg/kg)
plus IL-1β-ab (at 3mg/kg)

Figure 7

Cytokine antibodies attenuate the enhanced iNOS activity in the lung homogenates